# EUROPÄISCHE PATENTANMELDUNG

(11) **EP 4 134 113 A1**
(43) Veröffentlichungstag der Anmeldung: **15.02.2023**
(21) Anmeldenummer: 21190627.6
(22) Anmeldetag: 10.08.2021
(51) Int. Cl.: A61M 5/24, A61B 17/3203, A61M 5/30, A61M 5/20

(54) **APPLIKATOR MIT DOSIEREINHEIT**

(71) Anmelder: Erbe Elektromedizin GmbH, 72072 Tübingen (DE)
(72) Erfinder: FECH, Andreas, 72072 Tübeingen (DE); KLEBER, Frederik, 72827 Wannweil (DE); LINZENBOLD, Walter, 71034 Böblingen (DE)
(74) Vertreter: Rüger Abel Patentanwälte PartGmbB

(57) **Zusammenfassung**

Das erfindungsgemäße Instrument (10) weist eine Dosiereinheit (16) auf, die an den Handgriff des Instruments (10) angeschlossen ist. Dadurch wird die Länge der Zuleitung für die zu applizierende medizinisch wirksame Substanz gegenüber Lösungen deutlich reduziert, bei denen die Dosiereinheit Teil eines speisenden Geräts ist. Die Dosiereinheit (16) kann im Wesentlichen aus einem wiederverwendbaren Aktuator, der eine Antriebseinheit (25) umfasst und einer Einwegkartusche bestehen, die zum Beispiel, wie eine medizinische Spritze, einen Zylinder und einen Kolben umfasst. Die Einwegkartusche kann entweder unmittelbar vor dem Eingriff von dem Anmelder selbst befüllt werden oder herstellerseitig vorbefüllt sein. Sie wird einfach in das Instrument eingesetzt. Dazu wird die Dosiereinheit (16) in die offene, von dem Gehäuse (13) abgesetzte Position gebracht. Dies erfolgt indem der Rastschieber (37) betätigt und die Dosiereinheit in proximaler Richtung gezogen wird. Nach dem Einlegen der Kartusche (des Fluidbehälters (22)) wird die Dosiereinheit (16) in die geschlossene Position gebracht, d.h. an das Gehäuse (12) herangeschoben. Dabei wird eine fluiddichte Verbindung zwischen der Kartusche und dem zweiten Fluidkanal (47) geschaffen. Der Rastschieber (37) arretiert die Dosiereinheit (16) dabei in dieser Position.

## Beschreibung

Die Erfindung betrifft ein Instrument zur Applikation einer medizinischen Flüssigkeit in einem tierischen oder menschlichen Körper. Weiter betrifft die Erfindung ein Gerät zum Betrieb des Instruments sowie ein Verfahren zur Erzeugung eines Fluidstroms aus zwei verschiedenen Fluiden.

Zum Beispiel zur nadellosen Injektion von Medikamenten oder anderen medizinischen wirksamen Flüssigkeiten in das Gewebe von menschlichen oder tierischen Körpern werden Einrichtungen benutzt, die ein unter hohem Druck stehendes Fluid zur Durchbrechung der Haut des Patienten und nadellosen Erzeugung eines Stichkanals nutzen, in welchen dann ein unter niedrigerem Druck stehendes Behandlungsfluid eingeleitet wird.

Aus der EP 3 015 076 A1 ist ein Applikator bekannt, mit dem ein Behandlungsfluid, beispielsweise eine blutstillende Flüssigkeit, mittels Druckluft oder einem anderen unter Druck stehenden Gas zerstäubt und so auf zu behandelnde Flächen aufgebracht werden kann. Der Applikator umfasst einen Halter mit einem an eine Gasdruckquelle anschließbaren Fluidkanal, in dem ein Ventil angeordnet ist. Der Fluidkanal mündet an einer Zerstäuberdüse. An dem Applikator kann eine gewöhnliche Spritze befestigt werden. Der Nutzer kann, wenn er den Applikator in der Hand hält, mit einem Finger das Ventil für den Luftkanal freigeben und mit seinem Daumen den Kolben der Spritze betätigen, sodass die in der Spritze enthaltene Flüssigkeit zur Zerstäuberdüse geführt und dort als Spray ausgegeben wird.

Weiter offenbart die US 2013/0102957 A1 ein Instrument zur nadellosen Injektion einer Substanz in einen Körper. Das Instrument weist ein Reservoir zur Aufnahme der zu injizierenden Substanz auf, die über einen Fluidkanal an eine Injektionsdüse angeschlossen ist. Zum Ausstoß des Behandlungsfluids in einem kurzen scharfen Strahl ist einem in dem Reservoir angeordneten Kolben ein elektrodynamischer Antrieb zugeordnet, der an der Injektionsdüse einen Fluiddruckverlauf mit einer sehr steilen Vorderflanke erzeugt. Der Druckanstieg generiert einen scharfen Flüssigkeitsstrahl, der zur Durchbrechung der Haut geeignet ist. Nach dem steilen Druckanstieg fällt der Druck auf ein niederes Niveau zum Einleiten der Flüssigkeit in das Gewebe ab, um schließlich nach Ablauf des Betätigungszyklus wieder Null zu werden.

Zur nadellosen Injektion von Behandlungsflüssigkeiten schlägt außerdem die EP 3 714 926 A1 ein Instrument mit mehreren Fluidkanälen vor, die verschiedene Flüssigkeiten an eine Injektionsdüse heranführen. Insbesondere wird vorgesehen, einen zentralen Kanal für ein Injektionsfluid zum Öffnen eines Stichkanals vorzusehen, wobei der zentrale Kanal von einem zweiten Kanal umgeben ist, mit dem Behandlungsfluid an die Injektionsdüse herangeführt wird.

Das Instrument eignet sich prinzipiell gut zur Anwendung an Patienten. Dabei muss jedoch der für das Behandlungsfluid vorgesehene Kanal vollständig mit diesem Fluid gefüllt sein. Wird patientenspezifisches Behandlungsfluid verwendet, muss eine Fluidmenge an Behandlungsfluid bereitgestellt werden, die größer ist als die am Patienten zu applizierende Fluidmenge.

Dies gilt auch für das System nach der EP 3 040 101 B1**,** aus der ein Instrument mit zwei Fluidkanälen hervorgeht. Ein erster Kanal führt ein Injektionsfluid und ein zweiter Kanal ein Behandlungsfluid zu einer Applikationsdüse. Zur Speisung ist ein Gerät mit Druckspeicher und mehreren Ventilen vorgesehen, die insbesondere das Injektionsfluid mit einem vorgegebenen Druckregime an das Instrument liefern.

Es ist Aufgabe der Erfindung, ein verbessertes Konzept zur nadellosen Applikation von Behandlungsfluiden anzugeben.

Diese Aufgabe wird mit dem Instrument nach Anspruch 1, dem Gerät nach Anspruch 12 sowie dem Verfahren nach Anspruch 15 gelöst:

Das erfindungsgemäße Instrument dient zur Injektion einer medizinischen Flüssigkeit in einen tierischen oder menschlichen Körper, wobei das Instrument dazu eingerichtet ist, zwei verschiedene Fluide zu applizieren. Ein erstes Fluid dient zum nadellosen Öffnen eines Kanals in einem zur behandelnden Gewebe (Stichkanal) und wird mit einem Druck-Impuls ausgegeben, der an seiner Vorderflanke einen steilen Druckanstieg aufweist. Das Instrument weist außerdem eine Dosiereinheit auf, die ein Aufnahmefach für einen Fluidbehälter mit dem zweiten Fluid aufweist. Das zweite Fluid ist vorzugsweise ein Behandlungsfluid, beispielsweise eine medikamentöse Flüssigkeit, eine Zellsuspension oder irgendeine andere zur Behandlung eines menschlichen oder tierischen Körpers oder Gewebes dienende Flüssigkeit. Die Dosiereinheit weist eine Antriebseinheit auf, mittels derer der Fluidbehälter mit Druck beaufschlagbar oder gezielt im Volumen verkleinerbar ist. Zum Beispiel kann der Fluidbehälter von der Antriebseinheit gezielt so beeinflusst werden, dass sich das Volumen des Fluidbehälters vermindert, um dadurch das zweite Fluid sanft auszutreiben.

Nachdem der Fluidbehälter in der Dosiereinheit unmittelbar an dem Instrument angeordnet ist, sind Totvolumina minimiert. Verluste an Behandlungsflüssigkeit, die insbesondere bei Verwendung bei patientenindividueller Behandlungsflüssigkeit unerwünscht sind, werden so vermieden. Auch ist die Vorbereitung des Instruments wie auch die Vorbereitung des speisenden Geräts vereinfacht. Die Gefahr ist minimiert, Reste von Behandlungsflüssigkeit einer vorigen Behandlung in Kanälen, Pumpen oder Ventilen zu belassen und damit die Behandlungsflüssigkeit eines nachfolgenden Patienten zu kontaminieren.

Die Steuerung der Abgabe des ersten Fluids und des zweiten Fluids durch ein und dasselbe Gerät ermöglicht eine koordinierte und schonende Fluidabgabe. Beispielsweise kann das zweite Fluid lebende Zellen enthalten, die weder großen Druckschwankungen noch Scherkräften ausgesetzt werden dürfen und die sich mit dem erfindungsgemäßen Instrument unter Zuhilfenahme auch des erfindungsgemäßen Geräts besonders schonend applizieren lassen.

Die Dosiereinheit kann an dem Instrument lösbar befestigt sein. Dabei ist es insbesondere vorteilhaft, wenn die Dosiereinheit selbst keine mit dem ersten oder zweiten Fluid in Berührung kommenden Teil aufweist. Die Dosiereinheit kann beispielsweise aus einem Gehäuse mit einem Aufnahmefach für den Fluidbehälter und mit einer Antriebseinheit zur Kraft- oder Druckbeaufschlagung des Fluidbehälters bestehen. Ist die Dosiereinheit von dem Instrument abgenommen, kann das Instrument sterilisiert werden oder auch komplett ersetzt werden, ohne dass die Dosiereinheit ersetzt werden müsste. Die Dosiereinheit kann wiederverwendet werden. Bedarfsweise kann sie gereinigt und auch sterilisiert werden.

Das Instrument kann einen Schaft aufweisen, der an seinem distalen Ende einen abwinkelbaren Abschnitt aufweist, wobei zur Steuerung der Abwinkelung des Schafts an dem Gehäuse ein Bedienelement vorgesehen sein kann. Ein solches Instrument eignet sich beispielsweise zur Einführung in einen Harnleiter oder ein sonstiges Körperlumen und zum Injizieren einer lebende Zellen enthaltenden Trägerflüssigkeit zum Beispiel in einen Schließmuskel. Dazu kann der Fluidkanal an seinem distalen Ende eine strahlfokussierende Austrittsöffnung aufweisen.

In dem Gehäuse des Instruments kann eine Ventilanordnung vorgesehen sein, um in dem Fluidkanal einen Fluid-Hochdruckimpuls zu erzeugen. Diese Ventilanordnung kann jedoch auch Teil eines das Instrument speisenden Geräts sein.

Während das erste Fluid von dem Gerät über einen Schlauch an das Instrument herangeführt wird, kann die Dosiereinheit über eine elektrische Leitung mit dem Gerät verbunden sein. Die Steuerung der Dosiereinheit erfolgt dann über das Gerät, an das das Instrument angeschlossen ist.

Das Gehäuse des Instruments kann einen Fluidanschluss für den Fluidbehälter und einen mechanischen Steckanschluss für eine an der Dosiereinheit vorgesehene Steckvorrichtung aufweisen. Damit kann der Fluidbehälter beispielsweise in Gestalt einer Kartusche oder einer Spritze fluidmäßig mit dem Gehäuse verbunden werden und zur mechanischen Betätigung in die Dosiereinheit hinein ragen. Dabei ist es vorteilhaft, wenn die Steckvorrichtung und der Steckanschluss übereineinstimmende Betätigungsrichtungen aufweisen. Dies führt dazu, dass die Dosiereinheit beim Anstecken an das Gehäuse, wenn der Fluidbehälter in das Aufnahmefach eingelegt ist, zugleich den Fluidbehälter an den Fluidanschluss herandrückt und dabei eine Fluidverbindung herstellt. Der Fluidbehälter kann beispielsweise eine Kartusche oder irgendein anderer Behälter mit einem beweglichen Kolben sein.

Die Antriebseinheit der Dosiereinheit umfasst vorzugsweise einen Stellmotor, beispielsweise einen Schrittmotor, der über ein Getriebe auf den Fluidbehälter einwirkend angeordnet ist. Zum Beispiel ist das Getriebe ein Spindelgetriebe, das die Drehbewegung des Stellmotors in eine Linearbewegung umsetzt, mit der ein zu dem Fluidbehälter gehöriger Kolben gezielt und kontrolliert verschoben werden kann. Durch entsprechende Ansteuerung des Stellmotors kann der auf den Fluidbehälter einwirkende Abtrieb der Antriebseinheit eine definierte Strecke bewegt werden, wodurch eine definierte Flüssigkeitsmenge ausgegeben wird.

Zur Speisung des Instruments kann ein Gerät vorgesehen sein, das eine Steuereinheit aufweist, die dazu eingerichtet ist, zunächst das erste Fluid zur Abgabe desselben mit einem ersten Hochdruckimpuls und nachfolgend geringerem Druck zu veranlassen und das weiter dazu eingerichtet ist, nach dem Hochdruckimpuls die Dosiereinheit zur Erzeugung eines Niederdruckimpulses zu aktivieren, um das zweite Fluid mit geringem Druck über den Fluidkanal auszugeben.

Mit diesem Konzept wird mittels des ersten Fluids ein Kanal in dem Gewebe geöffnet. Im weiteren zeitlichen Verlauf wird dann nach Abklingen des ersten Druckimpulses die Behandlungsflüssigkeit (das zweite Fluid) in das erste Fluid geleitet, um schonend in das Gewebe eingeführt zu werden. Das Gerät kann das erste Fluid in einem Druckbehälter bereitstellen, aus dem es mit einem schnell schaltenden Ventil impulsweise ausgelassen wird. Außerdem kann das Gerät einen elektrischen Anschluss für die Dosiereinheit des Instruments haben.

Weitere Einzelheiten vorteilhafter Ausführungsformen der Erfindung sind Gegenstand, der Zeichnung oder der Beschreibung sowie von Unteransprüchen. Es zeigen:
Figur 1 ein erfindungsgemäßes Instrument und ein speisendes Gerät, in schematischer Prinzipdarstellung,
Figur 2 eine abgewandelte Ausführungsform des Instruments, in schematischer Prinzipdarstellung,
Figur 3 die Dosiereinheit des Instrument nach Figur 1 oder 2, in abschnittsweise längsgeschnittener Darstellung,
Figur 4 eine Schnittstelle zwischen dem Instrument und der Dosiereinheit, in längsgeschnittener Prinzipdarstellung,
Figur 5 einen Funktionsplan des Instruments und Geräts nach Figur 1 und 2 und
Figur 6 den idealisiert veranschaulichten Druckverlauf bei der Abgabe des ersten und des zweiten Fluids als Diagramm.

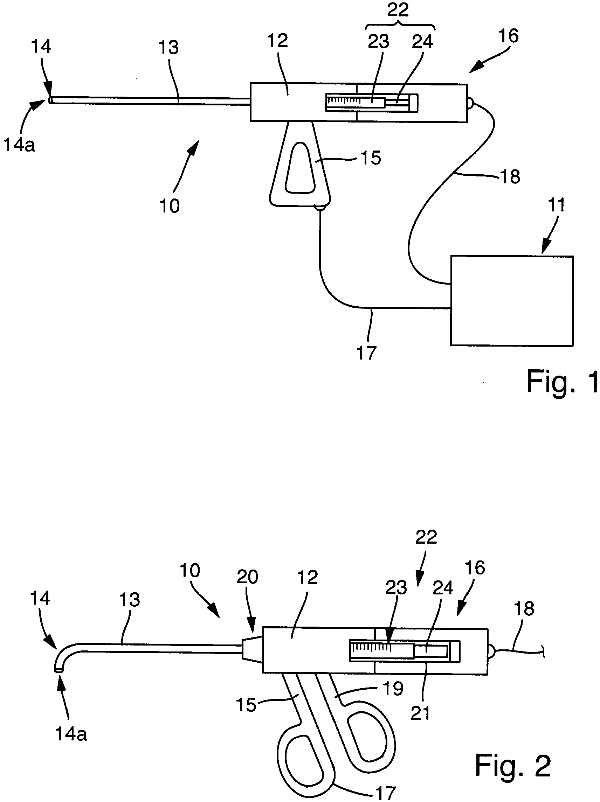

In Figur 1 ist ein Instrument 10 zur Injektion einer medizinischen Flüssigkeit in einen menschlichen oder tierischen Körper, insbesondere zur nadellosen Injektion einer solchen Flüssigkeit in das Gewebe des Körpers veranschaulicht. Das Instrument 10 ist an ein zu seiner Versorgung dienendes Gerät 11 angeschlossen, um das Instrument 10 mit Energie und/oder Medien, zum Beispiel Natriumchloridlösung, zu versorgen.

Das Instrument 10 weist ein Gehäuse 12 auf, von dem sich ein Schaft 13 in distaler Richtung bis zu seinem distalen Ende 14 erstreckt, an dem eine Injektionsöffnung 14a angeordnet ist, um einen Fluidstrahl auszulassen. An dem Gehäuse 12 kann ein Handgriff 15 zur manuellen Führung des Instruments 10 vorgesehen sein.

Das Instrument 10 ist außerdem mit einer Dosiereinheit 16 versehen, die dazu dient, zur Behandlung des Patienten zum Beispiel in sein Gewebe zu injizierendes medizinisches Fluid dosiert abzugeben. Die Dosiereinheit 16 kann an jeder geeigneten Stelle des Gehäuses 12, zum Beispiel an einer Seite, an seiner Oberseite, in seinem Handgriff 15 oder, wie in Figur 1 veranschaulicht, an seiner dem Schaft 13 gegenüber liegenden proximalen Rückseite angeordnet sein.

Das zur nadellosen Injektion von medizinischer Flüssigkeit in lebendes Gewebe vorgesehene Instrument 10 nutzt zur Öffnung eines Stichkanals in dem Gewebe ein erstes Fluid. Dieses ist eine mit Hochdruck zu applizierende Flüssigkeit, die von dem Gerät 11 über eine Fluidleitung 17 geliefert werden kann, die über die das Gerät 11 mit dem Instrument 10 verbunden ist.

Ein zweites Fluid, z.B. eine medizinisch wirksame Flüssigkeit, wird durch eine Dosiereinheit gezielt in das Gewebe des Patienten eingeleitet. Zur Ansteuerung der Dosiereinheit 16 kann das Gerät 11 mit dem Instrument 10 über eine elektrische Leitung 18 verbunden sein, die zum Beispiel durch ein zwei- oder mehradriges Kabel gebildet wird.

Zur Aktivierung des Instruments 10 kann an diesem ein Schalter vorgesehen sein, der dann über eine nicht weiter veranschaulichte Steuerleitung oder eine anderweitige Steuerverbindung mit dem Gerät 11 verbunden ist. Alternativ können andere Schalter Anwendung finden, wie zum Beispiel ein Fußschalter oder dergleichen.

Das in Figur 1 in besonders simpler Form veranschaulichte Instrument 10 kann weitere Funktionalitäten aufweisen, wie zum Beispiel ein abwinkelbares distales Ende 14, wobei das Abwinkeln zum Beispiel über einen Steuerhebel 19 bewirkt werden kann. Der Steuerhebel 19 kann in Nachbarschaft zu dem Handgriff 15 oder auch an anderer Stelle des Instruments 10 angeordnet sein. Außerdem ist es möglich, den Schaft 13 an dem Gehäuse 12 drehbar anzuordnen, wozu ein entsprechender Drehverbinder 20 vorgesehen sein kann. Im Übrigen gelten die Ausführungen zu dem Instrument 10 nach Figur 1 auch für das Instrument 10 nach Figur 2.

Die Dosiereinheit 16 des Instruments 10 weist ein Aufnahmefach 21 für einen Fluidbehälter 22 auf, der medizinische Behandlungsflüssigkeit enthält, beispielsweise eine Zellsuspension mit lebenden Zellen oder eine medikamentöse Flüssigkeit oder dergleichen. Das Aufnahmefach 21 kann sich bis in das Gehäuse 12 hinein erstrecken. Der Fluidbehälter 22 kann beispielsweise nach Art einer Kolbenspritze ausgebildet sein und dazu einen Zylinder 23 mit einem darin angeordneten Kolben 24 umfassen, dessen Kolbenstange aus dem Zylinder 23 heraus ragt.

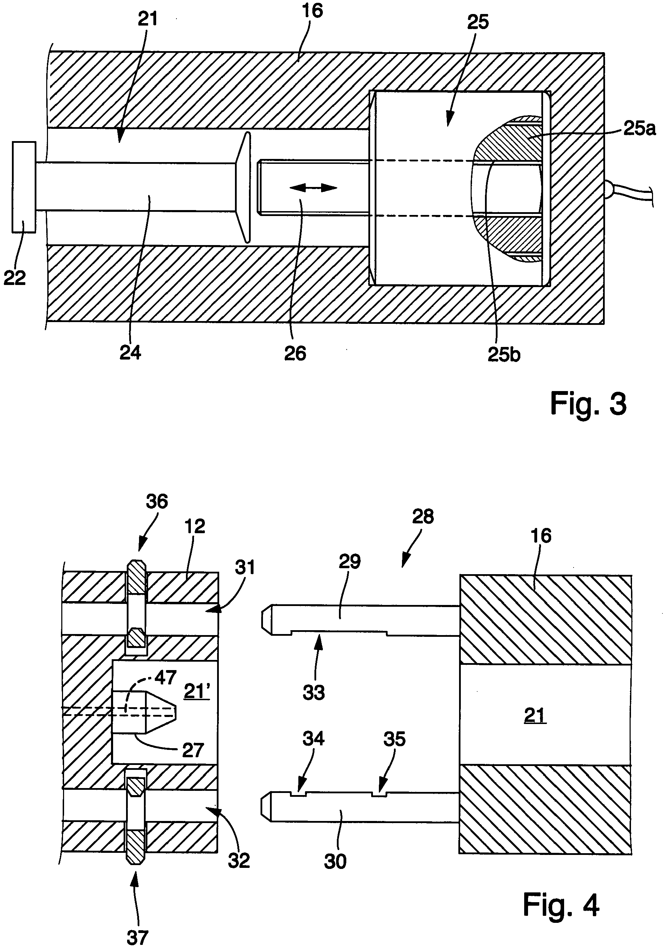

Wie Figur 3 erkennen lässt, umfasst die Dosiereinheit 16 eine Antriebseinheit 25, die zum Beispiel durch einen Motor mit drehendem Rotor 25a gebildet sein kann, der über ein Getriebe, beispielsweise ein Spindelhubgetriebe, 25b einen Stößel 26 linear bewegt. Dabei ist die Antriebseinheit 25 insbesondere darauf eingerichtet, den Stößel 26 graduell zu bewegen, um mit dem Stößel 26 über die Kolbenstange 24 den Kolben in dem Zylinder 22 (langsam) zu verschieben. Unter einer "langsamen" Verschiebung der Kolbenstange 24 werden dabei solche Bewegungen verstanden, die zu keinem für das Behandlungsfluid schädlichen Druckaufbau in dem Fluidbehälter 22 führen.

Wie Figur 4 veranschaulicht, erstreckt sich das Aufnahmefach 21 mit einem Abschnitt 21' in das Gehäuse 12 hinein. In dem Gehäuse 12 kann dann am Boden des Aufnahmefachs 21, 21' ein Anschlussstutzen 27 vorgesehen sein, der zum Anschluss des Fluidbehälters 22 geeignet ist. Der Anschlussstutzen 27 ist in Figur 4 lediglich schematisch veranschaulicht. Jedenfalls ist der Anschlussstutzen 27 auf den Anschluss des Fluidbehälters 22 abgestimmt, um eine fluiddichte Verbindung herbeizuführen, sobald der Fluidbehälter 22 in das Aufnahmefach 21 eingesetzt und auf den Anschlussstutzen 27 hin bewegt wird. Etwaige Verschlüsse oder Versiegelungen des Fluidbehälters 22 können dabei durchstoßen werden.

Die Dosiereinheit 16 ist insbesondere dafür vorgesehen, den Fluidbehälter 22 wechseln zu können. Dazu kann gemäß Figur 4 eine spezielle Schnittstelle zwischen dem Gehäuse 12 und der Dosiereinheit 16 vorgesehen sein, die insbesondere darauf eingerichtet sein kann, die Dosiereinheit 16 wahlweise an dem Gehäuse 12 anliegend zu halten, von dem Gehäuse 12 lediglich etwas abzurücken oder von dem Gehäuse 12 ganz abzunehmen.

Die Dosiereinheit 16 kann dazu an dem Gehäuse 12 mit einer Rastkupplung 28 befestigt sein. Die Rastkupplung 28 kann in einer einfachen und zweckmäßigen Ausführungsform zum Beispiel zwei Rastpins 29, 30 aufweisen, denen in dem Gehäuse 12 ausgebildete Rastöffnungen 31, 32 zugeordnet sind. Die Rastpins 29, 30 können z.B. einen runden Querschnitt oder auch jeden anderen geeigneten, z.B. ovalen oder polygonalen Querschnitt aufweisen und jeweils auf einem Teil ihrer Länge mit Ausnehmungen, d.h. z.B. Abflachungen 33, 34, 35 versehen sein. Der Rastpin 29 weist zum Beispiel eine lange Abflachung auf, während der Rastpin 30 mit zwei in axialem Abstand zueinander angeordneten kurzen nutartigen Abflachungen 34, 35 versehen sein kann. Den Abflachungen 33 bis 35 können entsprechende Rastschieber 36, 37 zugeordnet sein, um die Rastpins 29, 30 in den Rastöffnungen 31, 32 gezielt zu arretieren oder freizugeben. Dabei sind die Rastschieber 36, 37 sowie die Abflachungen 33 bis 35 in Verhältnis zueinander so angeordnet, dass ein Lösen des Rastschiebers 37 eine Bewegung der Dosiereinheit 16 von dem Gehäuse 12 weg innerhalb der axialen Länge der Abflachung 33 gestattet. Ist der Schieber 37 in die Abflachung 35 eingerastet, steht der Rastschieber 36 an der proximalen Flanke der Abflachung 33. Ist der Rastschieber 37 in die Abflachung 34 eingerastet, steht der Rastschieber 36 an der distalen Flanke der Abflachung 33. Erst durch Lösen beider Rastschieber 36, 37 lässt sich die Dosiereinheit 16 von dem Gehäuse 12 abnehmen. Die Abflachungen und Rastschieber sind jedoch nicht zwingend erforderlich.

Die Abnehmbarkeit der Dosiereinheit 15 von dem Instrument 10 ermöglicht es, das Instrument 10 als Einweginstrument oder als sterilisierbares Instrument auszubilden, wohingegen die Dosiereinheit 16 grundsätzlich dafür ausgelegt sein kann wiederverwendet zu werden. Der wiederverwendbare Teil kann bei einer abgewandelten Ausführungsform aber auch das Gehäuse, den Handgriff 15 oder Teile davon umfassen. Auch kann das Instrument insgesamt sterilisierbar ausgebildet sein.

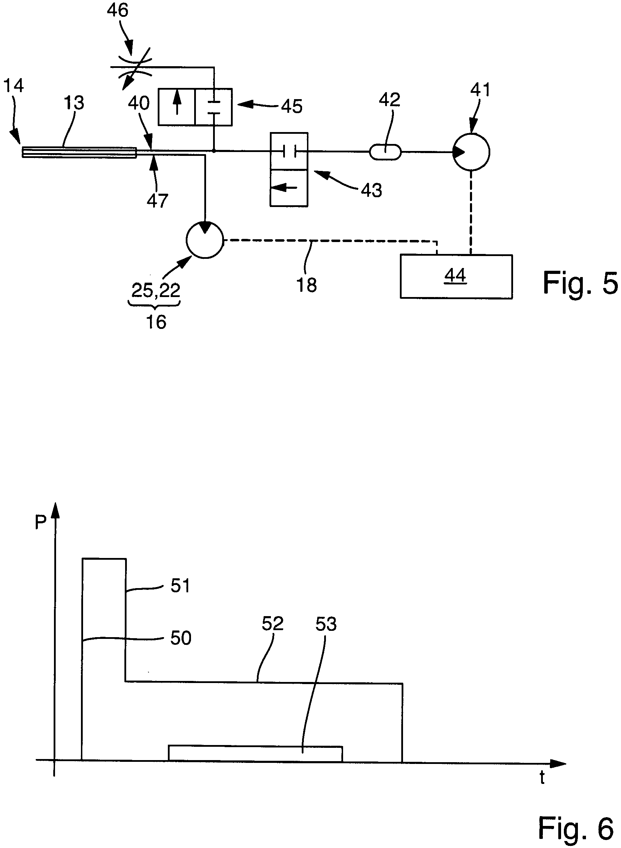

Figur 5 veranschaulicht die Funktionsbaugruppen und Komponenten des Instruments 10 und des Geräts 11 in ihrem Zusammenspiel. Es wird dabei darauf hingewiesen, dass einige oder alle der nachfolgend beschriebenen Komponenten und Baugruppen des Geräts 11 auch ganz oder teilweise in das Instrument 10 integriert sein können. Mindestens aber umfasst das Instrument 10 den Schaft 13, das Gehäuse 12 und die Dosiereinheit 16.

Das Instrument 10 weist einen ersten Fluidkanal 40 auf, der dazu vorgesehen ist, ein erstes Fluid an dem distalen Ende 14 mit so hohem Druck und als konzentrierten Stahl abzugeben, dass dieser ohne Zuhilfenahme einer Injektionsnadel in lebendes Gewebe eindringen und dort einen Kanal im Gewebe öffnen kann. Das erste Fluid ist vorzugs- und beispielsweise eine wässrige Natriumchloridlösung, wobei jedoch auch andere Fluide zur Anwendung kommen können. Das erste Fluid wird aus einem nicht weiter veranschaulichten Reservoir über eine Pumpe 41 mit Druck in einen Druckbehälter 42 geliefert, der über ein Ventil 43 an den ersten Fluidkanal 40 angeschlossen ist. Der Druckbehälter 42 kann auch allein durch die Zuleitung gebildet sein, so dass ein gesonderter Druckbehälter gegebenenfalls entfallen kann.

Das Ventil 43 wird von einer Steuereinrichtung 44 kontrolliert, d.h. gesteuert, und somit gezielt geöffnet oder geschlossen. Im Ruhezustand (zu Beginn der Applikation) ist das Ventil 43 geschlossen. Ein weiteres in Ruhezustand geschlossenes Ventil 45 zweigt von dem Fluidkanal 40 ab. Es dient zur Druckentlastung des Fluidkanals 40 und verbindet den Fluidkanal 40 wahlweise über eine Drossel 46 mit einem Abfluss. Das Ventil 45 und, falls gewünscht, auch die Drossel 46 können mit der Steuereinrichtung 44 verbunden sein, um von dieser gesteuert zu werden.

Die Dosiereinheit 16 beinhaltet als funktionelle Komponenten insbesondere die Antriebseinheit 25 und den als Kolbenpumpe dienenden Fluidbehälter 22. Dieser ist an einen zweiten Fluidkanal 47 angeschlossen, der sich von dem Anschlussstutzen 17 bis zu dem distalen Ende 14 des Schafts 13 erstreckt. Die Fluidkanäle 40, 47 können alternativ am distalen Ende 14 des Schafts 13, innerhalb des Schafts 13 oder innerhalb des Gehäuses 12 zusammengeführt sein.

Das insoweit beschriebene Instrument 10 und das Gerät 11 arbeiten insgesamt wie folgt:

Zur Inbetriebnahme und Vorbereitung einer Injektion von Behandlungsflüssigkeit in Gewebe wird zunächst die Dosiereinheit 16 mit der Behandlungsflüssigkeit bestückt. Dazu wird der Fluidbehälter 22 in das Aufnahmefach 21 eingesetzt. Zur Erleichterung oder Ermöglichung dieses Vorgangs fährt die Antriebseinheit 25 den Stößel 26 in proximale Position. Dies kann von der Steuereinheit 11 entsprechend veranlasst werden. Ein Bediener kann nun durch Betätigung mindestens des Rastschiebers 37 die Dosiereinheit 16 ein Stück von dem Gehäuse 12 abziehen und damit das Einlegen des Fluidbehälters 22 mit aus diesem herausragender Kolbenstange des Kolbens 24 in das Aufnahmefach 21 vornehmen. Ist dies geschehen, kann der Schieber 37, der bislang in die Abflachung 34 eingerastet ist, gelöst und die Dosiereinheit 16 an das Gehäuse 12 herangedrückt werden, bis der Schieber 37 in die Abflachung 35 einrastet. Dabei verbindet sich der Fluidbehälter 22 mit dem Anschlussstutzen 27, sodass der Fluidbehälter 22 nun mit dem zweiten Kanal 47 kommuniziert.

Des Weiteren aktiviert die Steuereinheit 44 die Pumpe 41, um den Druckbehälter 42 mit Injektionsfluid zu füllen und unter Druck zu setzen. Das Befüllen des Druckbehälters kann Teil des Applikationsvorgangs sein. Als vorbereitende Maßnahme kann außerdem der erste Fluidkanal 40 mit dem ersten Fluid, d.h. dem Injektionsfluid, gefüllt und somit entlüftet werden. Ist dies geschehen, ist das Instrument 10 betriebsbereit.

Zur Applikation der Behandlungsflüssigkeit wird nun der Schaft 13 in den Körper des Patienten, beispielsweise in ein Lumen desselben, eingeführt, zum Beispiel in den Harnleiter oder einen anderen natürlich vorgegeben Kanal. Mit dem distalen Ende 14 wird die Gewebepartie aufgesucht, in die die Behandlungsflüssigkeit zu applizieren ist. Ist dieser Ort erreicht, werden das erste Fluid (Injektionsflüssigkeit) und das zweite Fluid (Behandlungsflüssigkeit) nach den Schema gemäß Figur 6 abgegeben. Der Verlauf der in den beiden Fluiden herrschenden Drücke über der Zeit ist dort idealisiert veranschaulicht:

Zunächst wird das erste Fluid mit einer steilen Druckflanke 50 abgegeben, indem das Ventil 43 bei geschlossenem Ventil 45 öffnet. Damit entsteht an dem distalen Ende 14 ein scharfer Flüssigkeitsstrahl, der Gewebeschichten durchdringt und in das Gewebe eindringt. Nach Ablauf einer kurzen Zeit (einige Millisekunden) öffnet das Ventil 45, wodurch eine Absenkung des Drucks in dem Injektionsfluid mit der Rückflanke 51 erfolgt. Das erste Fluid wird dann weiter mit einem reduzierten Druck geliefert, wie in Figur 6 der Kurvenzug 52 andeutet. Währenddessen aktiviert die Steuereinheit 44 die Antriebseinheit 25, um, wie der Kurvenzug 53 idealisiert andeutet, mit geringem Druck ein zweites Fluid (Behandlungsflüssigkeit) an dem distalen Ende 14 abzugeben. Das Behandlungsfluid wird dann von dem Injektionsfluid mitgenommen und in das Gewebe eingetragen.

Die Injektionsbehandlung ist danach abgeschlossen. Die Antriebseinheit 25 wird deaktiviert und es werden zunächst das Ventil 43 und kurz darauf oder gleichzeitig das Ventil 45 geschlossen.

Der Injektionsvorgang kann in ähnlicher oder gleicher Weise an gleicher Stelle oder an anderer Stelle erneut vorgenommen werden bis der Fluidbehälter 22 geleert ist. Alternativ kann die Absenkung des Drucks durch die Reduzierung der Förderleistung der Pumpe realisiert werden. Das Ventil 45 wird in diesem Fall nur kurzzeitig zur schnellen Reduktion des Drucks, d.h. mit einer steilen Rückflanke, geöffnet. Im Anschluss an die Rückflanke wird das Ventil 45 geschlossen während das Ventil 43 noch geöffnet bleibt.

Zur Fortsetzung der Behandlung kann die Dosiereinheit 16 unter geeigneter Nutzung der Rastschieber 36, 37 von dem Gehäuse 13 ganz oder teilweise abgezogen werden, um den Fluidbehälter 22 zu ersetzen. Danach kann die Behandlung wie beschrieben weitergehen.

Das erfindungsgemäße Instrument 10 weist eine Dosiereinheit 16 auf, die an den Handgriff bzw. das Gehäuse 12 des Instruments 10 angeschlossen ist. Dadurch wird die Länge der Zuleitung für die zu applizierende medizinisch wirksame Substanz gegenüber Lösungen deutlich reduziert, bei denen die Dosiereinheit Teil eines speisenden Geräts ist. Die Dosiereinheit 16 kann im Wesentlichen aus einem wiederverwendbaren Aktuator, der eine Antriebseinheit 25 umfasst, und einer Einwegkartusche bestehen, die zum Beispiel, wie eine medizinische Spritze, einen Zylinder und einen Kolben umfasst. Die Einwegkartusche kann entweder unmittelbar vor dem Eingriff von dem Anwender selbst befüllt werden oder herstellerseitig vorbefüllt sein. Sie wird einfach in das Instrument eingesetzt. Dazu wird die Dosiereinheit 16 in die offene, von dem Gehäuse 13 abgesetzte Position gebracht. Dies erfolgt, indem der Rastschieber 37 betätigt und die Dosiereinheit in proximaler Richtung gezogen wird. Nach dem Einlegen der Kartusche (des Fluidbehälters 22) wird die Dosiereinheit in die geschlossene Position gebracht, d.h. an das Gehäuse 12 heran geschoben. Dabei wird eine fluiddichte Verbindung zwischen der Kartusche und dem zweiten Fluidkanal 47 geschaffen. Der Rastschieber 37 arretiert die Dosiereinheit 16 dabei in dieser Position.

### Bezugszeichen:

- 10: Instrument
- 11: Gerät
- 12: Gehäuse
- 13: Schaft
- 14: distales Ende des Schafts 13
- 14a: Injektionsöffnung
- 15: Handgriff
- 16: Dosiereinheit
- 17: Fluidleitung
- 18: elektrische Leitung
- 19: Steuerhebel
- 20: Drehverbinder
- 21: Aufnahmefach
- 21': Abschnitt des Aufnahmefachs
- 22: Fluidbehälter
- 23: Zylinder
- 24: Kolben mit Kolbenstange
- 25: Antriebseinheit
- 25a: Rotor
- 25b: Spindelhubgetriebe
- 26: Stößel
- 27: Anschlussstutzen
- 28: Rastkupplung / Steckvorrichtung
- 29, 30: Rastpin
- 31, 32: Rastöffnungen
- 33, 35: Abflachungen/Ausnehmungen
- 36, 37: Rastschieber
- 40: erster Fluidkanal
- 41: Pumpe
- 42: Druckbehälter
- 43: Ventil
- 44: Steuereinrichtung

- 45: Ventil
- 46: Drossel
- 47: zweiter Fluidkanal
- 50: steiler Druckanstieg
- 51: Rückflanke
- 52, 53: Kurvenzüge

## Patentansprüche

1. Instrument (10) zur Injektion einer medizinischen Flüssigkeit in einen tierischen oder menschlichen Körper,
mit einem Gehäuse (12), an dem ein Handgriff (15) zum Halten und Führen des Instruments (10) vorgesehen ist,
mit einem Schaft (13), der sich in distaler Richtung erstreckt und der mindestens einen Fluidkanal (40) aufweist,
mit einer Fluidleitung (17), die zur Verbindung mit einem speisenden Gerät (11) zur Versorgung des Instruments (10) mit einem unter Druck stehenden ersten Fluid versehen ist,
mit einer Dosiereinheit (16), die ein Aufnahmefach (21) für einen Fluidbehälter (22) mit einem zweiten Fluid und eine Antriebseinheit (25) zur Druckbeaufschlagung des Fluidbehälters (22) aufweist.

2. Instrument nach Anspruch 1, **dadurch gekennzeichnet, dass** die Dosiereinheit (16) an dem Gehäuse (12) lösbar befestigt ist.

3. Instrument nach Anspruch 2, **dadurch gekennzeichnet, dass** der Schaft (13) an seinem distalen Ende (14) einen abwinkelbaren Abschnitt aufweist und dass an dem Gehäuse (12) des Instruments (10) mindestens ein Bedienelement (19) zur Steuerung der Abwinkelung des Schafts (13) vorgesehen ist.

4. Instrument nach einem der vorstehenden Ansprüche,
**dadurch gekennzeichnet, dass** der Fluidkanal (40) an seinem distalen Ende eine strahlfokussierende Austrittsöffnung aufweist.

5. Instrument nach einem der vorstehenden Ansprüche,
**dadurch gekennzeichnet, dass** das Instrument (10) oder das Gerät (11) eine Ventilanordnung (43, 45) aufweist, um in dem Fluidkanal (40) einen Hochdruckimpuls (50) zu erzeugen.

6. Instrument nach einem der vorstehenden Ansprüche,
**dadurch gekennzeichnet, dass** die Dosiereinheit (16) eine elektrische Leitung (18) aufweist, die an eine Steuereinrichtung (44) anschließbar ist.

7. Instrument nach einem der vorstehenden Ansprüche,
**dadurch gekennzeichnet, dass** das Gehäuse (12) einen Fluidanschluss (27) für den Fluidbehälter (22) und einen mechanischen Steckanschluss (31, 32) für eine an der Dosiereinheit (16) vorgesehene Steckvorrichtung (28) aufweist.

8. Instrument nach Anspruch 7, **dadurch gekennzeichnet, dass** der Fluidanschluss (27) und die Steckvorrichtung (28) übereinstimmende Betätigungsrichtungen aufweisen.

9. Instrument nach einem der vorstehenden Ansprüche,
**dadurch gekennzeichnet, dass** der Fluidbehälter (22) einen beweglichen Kolben (24) aufweist.

10. Instrument nach den Ansprüchen 8 und 9, **dadurch gekennzeichnet, dass** die Bewegungsrichtung des Kolbens (24) mit der Betätigungsrichtung übereinstimmt.

11. Instrument nach einem der vorstehenden Ansprüche,
**dadurch gekennzeichnet, dass** die Antriebseinheit (25) einen Stellmotor umfasst, der über ein Getriebe auf den Fluidbehälter (22) einwirkend angeordnet ist.

12. Gerät (11) zum Betrieb des Instruments (10) nach einem der vorstehenden Ansprüche,
mit einer Steuereinheit (44), die dazu eingerichtet ist,
- Zunächst das erste Fluid zur Abgabe desselben zunächst mit einem ersten Hochdruckimpuls (50) und nachfolgend mit geringem Druck über den Fluidkanal (40) zu veranlassen und
- Nach dem Hochdruckimpuls (50) die Dosiereinheit (16) zur Erzeugung eines Niederdruckimpulses (53) zu aktivieren, um das zweite Fluid mit geringem Druck über den Fluidkanal (47) auszugeben.

13. Gerät nach Anspruch 12, **dadurch gekennzeichnet, dass** das Gerät (11) zur Erzeugung des Hochdruckimpulses (50) einen Druckspeicher (42) aufweist, der mittels einer Pumpe (41) mit dem ersten, unter hohem Druck stehenden Fluid füllbar ist und der ein Freigabeventil (43) mit dem Instrument (10) verbindbar ist.

14. Gerät nach einem der Ansprüche 12 oder 13, **dadurch gekennzeichnet, dass** das Gerät (11) einen Fluidausgang zum Anschluss der Fluidleitung (17) des Instruments (10) nach Anspruch 1 sowie einen elektrischen Anschluss für die Dosiereinheit (16) aufweist.

15. Verfahren zur Erzeugung eines Fluidstroms aus zwei verschiedenen Fluiden,
bei dem ein erstes Fluid in einem Druckspeicher (42) unter einem ersten Druck bereitgehalten und über ein plötzlich öffnendes Ventil (43) in eine Fluidleitung (40) eingeleitet wird und
bei dem ein zweites Fluid in einem Gefäß (22) drucklos bereitgehalten und nach dem Öffnen des Ventils (43) durch Verminderung des Volumens des Gefäßes (22) mit einem zweiten Druck in die Fluidleitung (47) eingeleitet wird, der niedriger ist, als der erste Druck,
wobei zur Verminderung des Volumens des Gefäßes (22) eine Antriebseinheit (25) mit einem Stellmotor vorgesehen ist und dass die Antriebseinheit (25) und das Ventil (43) von einer gemeinsamen Steuereinrichtung (44) gesteuert sind.
